# EUROPEAN PATENT SPECIFICATION

(11) **EP 2 212 338 B1**
(45) Date of publication and mention of the grant of the patent: **10.07.2013**
(21) Application number: 08841088.1
(22) Date of filing: 24.10.2008
(51) Int. Cl.: C07F 9/38, A61P 35/00

(54) **POLYMORPHIC FORMS OF IBANDRONATE SODIUM AND PROCESSES FOR PREPARATION THEREOF**
POLYMORPHE FORMEN VON IBANDRONAT-NATRIUM UND VERFAHREN ZU DEREN HERSTELLUNG
FORMES POLYMORPHES D'IBANDRONATE DE SODIUM ET LEURS PROCÉDÉS DE PRÉPARATION

(30) Priority: 26.10.2007 EP 07119358; 20.05.2008 EP 08156592
(43) Date of publication of application: 04.08.2010
(73) Proprietor: Chemo Ibérica, S.A., 08028 Barcelona (ES)
(72) Inventor: VENTIMIGLIA, Gianpiero, I-72021 Francavilla Fontana (br) (IT); MAGRONE, Domenico, I-20128 Milano (IT); CASTALDI, Graziano, I-28072 Briona (no) (IT)
(74) Representative: ZBM Patents
(86) International application number: PCT/EP2008/064401
(87) International publication number: WO 2009/053445

(56) References cited:
- WO-A-2006/024024
- WO-A-2006/081962
- WO-A-2007/074475
- WO-A-2008/060609
- HALEBLIAN J ET AL: "PHARMACEUTICAL APPLICATIONS OF POLYMORPHISM" JOURNAL OF PHARMACEUTICAL SCIENCES, AMERICAN PHARMACEUTICAL ASSOCIATION. WASHINGTON, US, vol. 58, no. 8, August 1969 (1969-08), pages 911-929, XP009058367 ISSN: 0022-3549
- MCCRONE W C: "POLYMORPHISM", PHYSICS AND CHEMISTRY OF THE ORGANIC SOLID STATE, XX, XX, vol. 2, 1 January 1965 (1965-01-01), pages 725-767, XP001156955,
- CAIRA M R: "CRYSTALLINE POLYMORPHISM OF ORGANIC COMPOUNDS", TOPICS IN CURRENT CHEMISTRY, SPRINGER, BERLIN, DE LNKD- DOI:10.1007/3-540-69178-2_5, vol. 198, 1 January 1998 (1998-01-01), pages 163-208, XP001156954,
- BRITTAIN: "X-RAY DIFFRACTION III: PHARMACEUTICAL APPLICATIONS", SPECTROSCOPY, ELSEVIER, AMSTERDAM, NL, vol. 16, 1 January 2001 (2001-01-01), pages 142-150, XP009082169, ISSN: 0712-4813

## Description

### Field of the invention

The present invention relates to novel polymorphic forms of Ibandronate sodium, to processes for preparing them, to pharmaceutical compositions containing them, as well as their use.

### Background of the invention

Ibandronate sodium is a third-generation nitrogen-containing biphosphonate derivative characterized by an aliphatic tertiary amine side chain.

Ibandronate sodium is one of the most potent anti-resorptive drugs that directly inhibit osteoclast activity, presenting a pharmacologic alternative for controlling hypercalcemia. Ibandronate sodium binds to hydroxyapatite in calcified bones, rendering them resistant to hydrolytic dissolution by phosphatases, thereby inhibiting both normal and abnormal bone resorption. This drug increases bone mass and decreases the risk of fractures and is therefore particularly adapted to bone diseases and calcium metabolic disorders such as osteoporosis, osteolysis, Paget's disease, Bechterew's disease, bone metastases, urolithiasis, heterotropic ossifications, rheumatoid arthritis, osteoarthritis and degenerative arthrosis.

Ibandronate sodium is a compound of Formula (I)

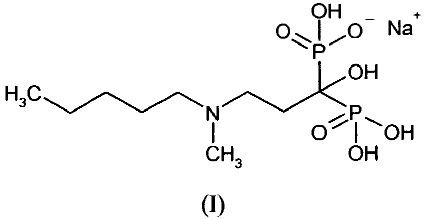

[3-(N-methyl-N'-pentyl)-amino-1-hydroxypropane-1,1-diphosphonic acid monosodium salt], which is disclosed in U.S. Patent 4,927,814. It is marketed under the name of Boniva® for the treatment of bone disorders such as hypercalcaemia of malignancy, osteolysis, Paget's disease, osteoporosis, and metastatic bone disease. Ibandronate sodium is also marketed in Europe under the name of Bondronat®.

The present invention relates to novel polymorphic forms of Ibandronate sodium and processes for preparation thereof.

Polymorphism is the property of some molecules and molecular complexes to assume more than one crystalline or amorphous form in the solid state. Substances are known which only appear in a single crystal form; in addition, however, there are also substances which can form two, three or even more polymorphic crystal modifications. In general, polymorphism is caused by the ability of the molecule of a substance to change its conformation or to form different inter molecular and intramolecular interactions, particularly hydrogen bonds, which is reflected in different atom arrangements in the crystal lattices of different polymorphs. Accordingly, polymorphs are distinct solids sharing the same molecular Formula, having distinct advantageous and/or disadvantageous physical properties compared to other forms in the polymorph family.

The morphology and polymorphology of organo-chemical active substances is of great importance to the chemical and pharmaceutical development thereof.

The relevant polymorphism of an organo-chemical substance is always unpredictable in respect of the number of crystal modifications, the stability thereof and their behaviour in a living organism.

The different polymorphs of a substance possess different energies of the crystal lattice and, thus, they show different physical properties of the solid state such as form, density, melting point, colour, stability, dissolution rate, milling facility, granulation, compacting etc. These differences in morphology and polymorphism may have drastic effects on the flowability of the milled solid (flowability affects the ease with which the material is handled during processing into a pharmaceutical product; when particles of the powdered compound do not flow past each other easily, a formulation specialist must necessitate the use of glidants), development, transport stability and storage stability of individual administration forms, on the ability to produce different administration forms, on their application, on the solubility in polar or non-polar, protic or aprotic solvents, on solubility in aqueous solution, on solubility in the gastric juices, on solubility in blood serum, and finally on bio-availability. The rate of dissolution of an active ingredient in a patient's stomach fluid can have therapeutic consequences since it imposes an upper limit on the rate at which an orally-administered active ingredient can reach the patient's bloodstream. The rate of dissolution is also a consideration in formulating syrups, elixirs and other liquid medicaments. Other important properties of polymorphic forms relate to the ease of processing the form into pharmaceutical dosages, as the tendency of a powdered or granulated form to flow and the surface properties that determine whether crystals of the form will adhere to each other when compacted into a tablet. The polymorphic form may give rise to thermal behavior different from that of the amorphous material or another polymorphic form. Thermal behavior is measured in the laboratory by such techniques as capillary melting point, Differential Scanning Calorimetry (DSC) and can be used to distinguish some polymorphic forms from others. A particular polymorphic form may also give rise to distinct spectroscopic properties that may be detectable by X-Ray Powder Diffraction (XRPD).

Generally, the crystalline solid has improved chemical and physical stability over the amorphous form, and forms with low crystallinity. They can also exhibit improved solubility, hygroscopicity, bulk properties, and/or flowability. The same also applies in respect of the physical and chemical properties of Ibandronate sodium.

It has been found that Ibandronate sodium may exist in various polymorphs.

According to the Patent Applications WO-A-2006081962 and WO-A-2006081963, two crystalline form of Ibandronate sodium, monohydrate salt, herein designated as Forms A and B, were disclosed.

The Patent Application WO-A-2006024024 relates to solid amorphous and crystalline forms of Ibandronate sodium (herein designated as crystalline Forms C, D, E, F, G, H, J, K, K2, K3, Q, Q1, Q2, Q3, Q4, Q5, Q6, QQ, R, S, T) and processes for preparation thereof, where some forms of them are solvates. Form C can exist as monoethanolate, Form E can exist as hemibutanolate, Form R and S can exist as hemiethanolate.

The Patent Application WO-A-2007074475 relates to novel polymorphic forms of Ibandronate sodium monohydrate and processes for their preparation. The novel crystalline forms are designated as Form I and Form II, and the amorphous Ibandronate sodium monohydrate as Form III.

The discovery of novel polymorphic forms of a pharmaceutically useful compound provides a new opportunity to improve the performance characteristics of a pharmaceutical product. It enlarges the repertoire of materials that a formulation scientist has available for designing a pharmaceutical dosage form, or a drug with a targeted release profile, or other desired characteristics, such as flowability and suitable rate of dissolution in aqueous fluid. Therefore, the preparation of novel crystalline forms of Ibandronate sodium is desirable.

An object of the present invention is to provide novel polymorphic crystalline forms of Ibandronate sodium, processes for preparing them, pharmaceutical compositions containing them, as well as their use.

### Summary of the invention

The present invention provides Ibandronate sodium in new crystalline forms designated as Form ε and Form ζ (zeta).

In one aspect, the invention provides a novel polymorphic crystalline form of Ibandronate sodium, herein designated as Form ε. The crystalline Form ε of Ibandronate sodium is characterized by an XRPD pattern wherein the most prominent peaks are as follows:

| 2θ (degrees) (± 0.2°) | I/Imax (%) |
|---|---|
| 4,9 | 100,0 |
| 6,0 | 8,8 |
| 9,9 | 0,5 |
| 11,0 | 0,5 |
| 13,6 | 0,4 |
| 14,0 | 0,5 |
| 16,0 | 0,2 |
| 17,1 | 0,8 |
| 18,6 | 0,9 |
| 19,4 | 0,7 |
| 19,9 | 1,2 |
| 22,1 | 0,4 |
| 23,8 | 0,4 |
| 24,7 | 0,5 |
| 25,9 | 0,7 |
| 26,5 | 0,5 |
| 28,0 | 0,5 |
| 28,4 | 0,6 |
| 30,3 | 0,7 |
| 34,9 | 0,3 |
| 36,3 | 0,7 |

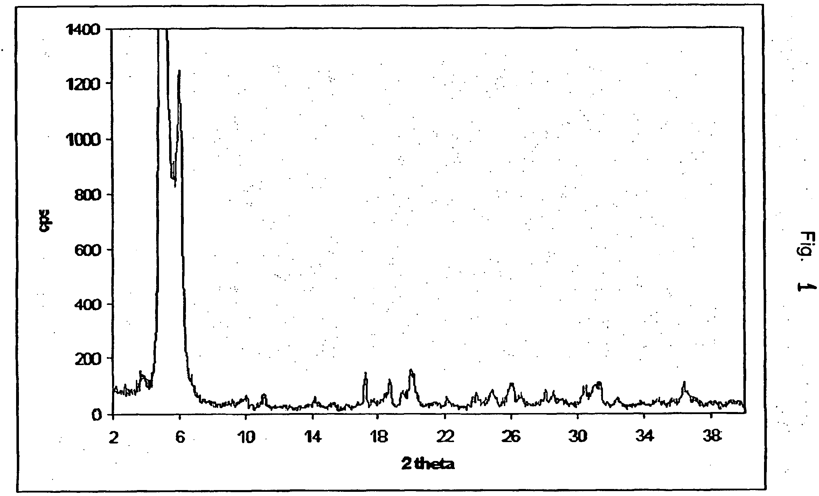

having an X-ray powder diffraction pattern as shown in Figure 1, wherein said crystalline form is an hemisolvate of 2-propanol and a hemihydrate.

In another aspect, the invention provides a novel polymorphic crystalline form of Ibandronate sodium, herein designated as Form zeta. The crystalline Form zeta of Ibandronate sodium is characterized by an XRPD pattern comprising the following peaks:

| 2θ (degrees) (± 0.2°) | I/Imax (%) |
|---|---|
| | |
| 6,2 | 100 |
| 12,3 | 1 |
| 14,5 | 1 |
| 15,1 | 1 |
| 16,9 | 2 |
| 17,3 | 2 |
| 18,0 | 1 |
| 19,6 | 1 |
| 20,0 | 1 |
| 21,1 | 1 |
| 21,5 | 1 |
| 24,7 | 2 |
| 25,9 | 5 |
| 26,7 | 2 |
| 27,6 | 1 |
| 29,2 | 2 |
| 31,0 | 4 |
| 34,6 | 1 |
| 37,2 | 3 |

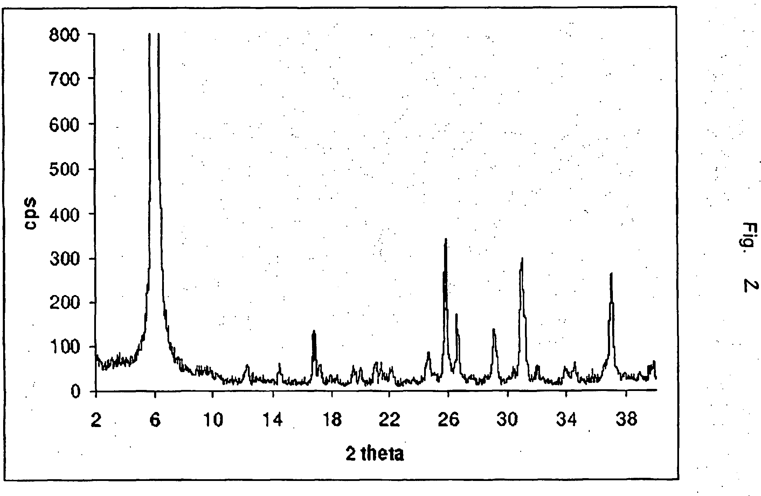

characterized by an X-ray powder diffraction pattern as shown in Figure 2 and wherein said form is a hemihydrate.

Form ε is a stable solvate form, allowing the isolation and purification of ibandronate sodium in high yields. Hemihydrate Form zeta is a stable crystalline form, which may be obtained from the corresponding solvate form without degenerative phenomena. The new form zeta resulted to be more useful for formulation. Solvate ε is useful for preparing said form.

In another aspect, the invention provides a process for preparing the crystalline Form ε of Ibandronate sodium comprising the steps of:
a) suspending ibandronate sodium in a mixture of water and 2-propanol;
b) heating the suspension in order to obtain a clear solution;
c) cooling the solution in order to crystallize a solid;
d) recovering the crystalline form.

In another aspect, the invention provides a process for preparing the crystalline Form zeta of Ibandronate sodium comprising the step of refluxing Form ε in acetone with 5-6% (v/v) of water. In another aspect, the invention provides a process for preparing the crystalline Form zeta of Ibandronate sodium comprising:
a) refluxing in 2-propanol a crystalline Form ε,
b) cooling the system to precipitate a solid,
c) isolating the solid,
d) drying at a temperature comprised in the range of 135-140°C under reduced pressure to render Form zeta.

In another aspect, the invention provides a crystalline Form zeta of Ibandronate sodium for use as a medicament.

In another aspect, the invention provides a pharmaceutical composition comprising the crystalline Form zeta of Ibandronate sodium, optionally together with at least one pharmaceutically acceptable excipient.

### Brief Description of the Drawings

Figure 1 provides a characteristic XRPD pattern of crystalline Form ε of Ibandronate sodium.
Figure 2 provides a characteristic XRPD pattern of crystalline Form zeta of Ibandronate sodium.
Figure 3 provides a characteristic DSC curve of crystalline Form zeta of Ibandronate sodium. It shows a first transition at 172 °C, with an overall enthalpy (endo) of 9.0 J/gram and a second transition with two close peaks at 183 and 188 °C, with an overall enthalpy (endo) of 32 J/gram.

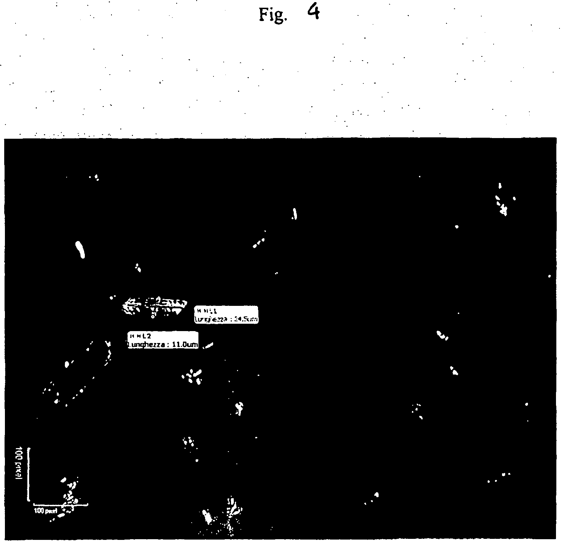

Figure 4 provides a microscopic photograph of crystals of Form zeta of Ibandronate sodium. Measured crystal has the following dimensions 34.5 µm x 11.0 µm.

### Detailed description of the invention

All terms as used herein in this application, unless otherwise stated, shall be understood in their ordinary meaning as known in the art. Other more specific definitions for certain terms as used in the present application are as set forth below and arc intended to apply uniformly through-out the specification and claims unless an otherwise expressly set out definition provides a broader definition.

The term "about" encompasses the range of experimental error that may typically occur in a measurement.

The term "excipient" herein means any substance, not itself a therapeutic agent, used as a carrier or vehicle for delivery of a therapeutic agent to a subject or added to a pharmaceutical composition to improve its handling or storage properties or to permit or facilitate formation of a dose unit of the composition into a discrete article such as a tablet, capsule, pill, powder, granule, pellet, lozenge, pastille, elixir, syrup, solution, suspension, emulsion, drop, lotion, spray, tincture, cream, ointment, gel, unguent, suppository and transdermal devices for oral, enteral, parenteral or topical administrations.

The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

We have now surprisingly found that Ibandronate sodium may be prepared in a manner that result in novel polymorphic forms that are until now unknown. The novel polymorphic forms have been designated as Forms ε and ζ (zeta) of Ibandronate sodium. These novel forms of Ibandronate sodium arc found to be stable at room temperature, reproducible and suitable for pharmaceutical preparations. They can be prepared with efficient and economic processes particularly suited to large-scale preparation.

XRPD analysis was used to characterize polymorphic forms of Ibandronate sodium.

XRPD analysis was performed on a APD 2000 Ital Structures diffractometer at room temperature, using a CuKα tube (40 kV, 30 mA, λ = 1.5418 Å) as the X-ray source. Data collection was made in 2θ step scan mode, at a scan speed of 0.02°/s (of 0.04% for Form zeta) in the range of 3° to 40° in 2θ. Approximately 100 mg samples were accurately grinded and placed on an aluminium sampler.

DSC (Differential Scanning Calorimetry) was performed with sample held in a vented aluminium crucible and analyzed at a heating rate of 10 °C per minute.

The crystalline forms of the present invention may be recovered by methods well known to those skilled in the art for the separation of the crystallized solid from the mother liquor, for example by filtration, with or without the assistance of pressure and/or vacuum, or by centrifugation, or by decantation. The collected solid is washed with at least a solvent and dried by conventional means.

The novel crystalline Form ε of Ibandronate sodium has an XRPD pattern as depicted in Figur 1. Most prominent peaks of XRPD pattern of Form ε are as follows:

| 2θ (degrees) (± 0.2°) | I/Imax (%) |
|---|---|
| 4,9 | 100,3 |
| 6,0 | 8,8 |
| 9,9 | 0,5 |
| 11,0 | 0,5 |
| 13,6 | 0,4 |
| 14,0 | 0,5 |
| 16,0 | 0,2 |
| 17,1 | 0,8 |
| 18,6 | 0,9 |
| 19,4 | 0,7 |
| 19,9 | 1,2 |
| 22,1 | 0,4 |
| 23,8 | 0,4 |
| 24,7 | 0,5 |
| 25,9 | 0,7 |
| 26,5 | 0,5 |
| 28,0 | 0,5 |
| 28,4 | 0,6 |
| 30,3 | 0,7 |
| 34,9 | 0,3 |
| 36,3 | 0,7 |

In another aspect, the invention provides a process for the preparation of Form ε of Ibandronate sodium comprising the steps of:
a) suspending Ibandronate sodium in a mixture of water and 2-propanol. Preferably, water and 2-propanol are in a ratio of about 1:3 to about 1:5 volume/volume. Preferably, Ibandronate sodium is in a ratio of about 1:4 to about 1:6 weight/volume with respect to water;
b) heating the suspension in order to dissolve completely the Ibandronate sodium. Preferably, the mixture is heated to a temperature comprised in a range of about 80 to about 100 °C;
c) Cooling the solution to a suitable temperature in order to crystallize a solid. A suitable temperature is comprised in a range of about 5 °C to about 25 °C; preferably, the temperature is comprised in a range of about 10 °C to about 20 °C; more preferably, the temperature is about 15 °C.
d) recovering the crystalline form.

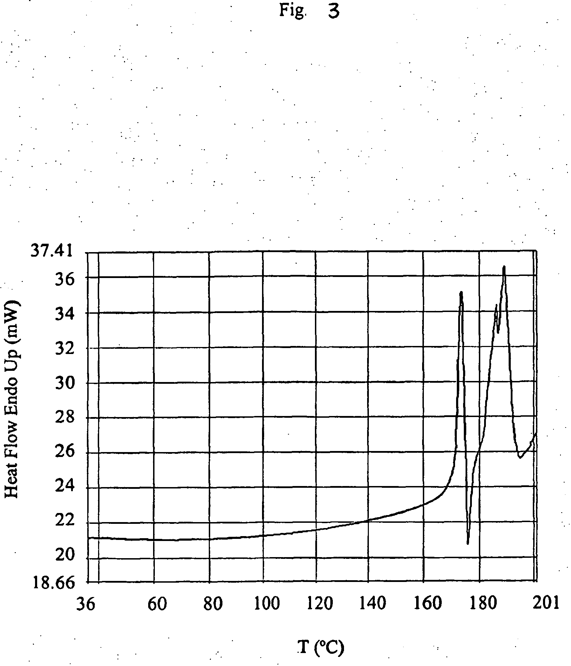

The novel crystalline Form zeta has an XRPD pattern as depicted in Figure 2. Form zeta may be isolated with a water content of 2.4-2.8% (determined by Karl Fischer method), which corresponds to a hemihydrate. When exposed to atmospheric humidity, Form zeta may uptake water to a content of 3.5-3.8%, showing no change in its XRPD pattern. A probable explanation for this behavior is that the crystal structure may accommodate additional loosely bound water. Form zeta shows a DSC curve as depicted in Figure 3. The DSC of Form zeta is further characterized by an endothermic peak at about 172 °C and two close endothermic peaks at about 183 °C and 188 °C.

Form zeta has shown stability in preliminary stability studies. Form zeta crystallizes in the form of little needles and plates. Advantageously, due to its morphology, Form zeta may be easier to filter and safer to handle and store.

Crystalline Form zeta of Ibandronate sodium may be obtained by a process comprising the step of refluxing Form ε in acetone with 5-6 % (v/v) of water. The system is preferably allowed to cool to isolate a solid. The solid may be dried under reduced pressure to render Form zeta.

Crystalline Form zeta of Ibandronate sodium may also be obtained by a process comprising:
a) refluxing in 2-propanol a crystalline Form ε,
b) cooling the system to precipitate a solid,
c) isolating the solid,
d) drying at a temperature comprised in the range of 135-140°C under reduced pressure to render Form zeta.

One skilled in the art will appreciate that by adjusting concentration, temperature and time the yield of crystalline Forms ε and zeta of Ibandronate sodium may be optimized.

As mentioned above the compound of the invention has useful therapeutic properties.

In another aspect, the invention provides crystalline Form zeta for use as a medicament.

The Form zeta of Ibandronate sodium may be administered per se or, preferably, as a pharmaceutical composition.

In another aspect, the invention provides a pharmaceutical composition comprising the crystalline Form zeta, optionally together with at least one pharmaceutically acceptable excipient.

The polymorphic forms may be used as single components or mixtures.

Excipients include, by way of illustration and not limitation, diluents, fillers, agglutinants, disintegrants, disintegration inhibitors, absorption accelerators, binders, carriers, suspending/dispersing agents, film formers/coatings, adhesives, antiadherents, wetting agents, lubricants, glidants, preservatives, sorbents, surface active agents, substances added to mask or counteract a disagreeable taste or odor, flavorings, colorants, fragrances, aromatising agents, sweeteners and substances added to improve appearance of the composition.

A person skilled in the art is aware of a whole variety of such excipient compounds suitable to formulate a pharmaceutical composition. The choice of excipient will to a large extent depend on factors such as the particular mode of administration, the effect of the excipient on solubility and stability, and the nature of the dosage form.

A crystalline Form zeta of Ibandronate sodium, together with a conventionally employed excipient, may be placed into the form of pharmaceutical compositions and unit dosages thereof, and in such form may be employed as solids, such as tablets, capsules, pills, powders, granules, pellets, lozenges, pastilles, or liquids, such as solutions, suspensions, emulsions, drops, lotions, sprays, tinctures, syrups, elixirs, or capsules filled with the same, all for oral use, or in the form of sterile injectable solutions for parenteral (including subcutaneous, intramuscular, and intravenous) use, or in the form of suppositories for rectal use, or in the form of creams, ointments, gels, unguents for topical use and other forms suitable for the inhalatory or transdermal administrations. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

Pharmaceutical compositions containing a crystalline Form zeta of Ibandronate sodium can be prepared in a manner well known in the pharmaceutical art and comprise at least one active compound. Generally, the compounds of this invention are administered in a pharmaceutically effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

The pharmaceutical compositions of the present invention can be administered by a variety of routes including oral, rectal, parenteral (including subcutaneous, intravenous, intramuscular), topical, transdermal, ophtalmic and intranasal. The compositions for oral administration can take the form of bulk liquid solutions or suspensions, or bulk powders. More commonly, however, the compositions arc presented in unit dosage forms to facilitate accurate dosing. Typical unit dosage forms include prefilled, premeasured ampoules or syringes of the liquid compositions or pills, tablets, capsules, powders, granules, pellets, lozenges, pastilles, suppositories or the like in the case of solid compositions. In such compositions, a crystalline Form zeta of Ibandronate sodium is usually a minor component (from about 0.1 to about 50% by weight or preferably from about 1 to about 40% by weight) with the remainder being various vehicles or carriers and processing aids helpful for forming the desired dosing form.

Solid forms may include, for example, any of the following ingredients, or compounds of a similar nature: a binder, such as cellulose-derived materials like powdered cellulose, microcrystalline cellulose, microfine cellulose, methyl cellulose, ethyl cellulose, hydroxyethyl cellulose, hydroxypropyl cellulose, hydroxypropylmethyl cellulose, carboxymethyl cellulose salts and other substituted and unsubstituted celluloses, gum tragacanth or gelatine; an excipient, such as starch or lactose, a disintegrating agent, such as alginic acid, primogel, or corn starch; a lubricant, such as magnesium stearate, hydrogenated castor oil, polyethylene glycol; a glidant, such as colloidal silicon dioxide; a sweetening agent, such as sorbitol, sucrose, aspartame or saccharin; or a flavoring agent, such as maltol, vanillin, menthol, citric acid, pepper-mint, methyl salicylate, or orange flavoring.

Liquid forms suitable for oral administration may include a suitable aqueous or nonaqueous vehicle with buffers, suspending and dispersing agents, colorants, flavors and the like.

Injectable compositions are typically based upon injectable sterile saline or phosphate-buf-fered saline or other injectable carriers known in the art. As above mentioned, a crystalline Form zeta of Ibandronate sodium in such compositions is typically a minor component, with the remainder being the injectable carrier and the like.

The above described components for orally administered or injectable compositions are merely representative. Further materials as well as processing techniques and the like are set out in Part 5 of Remington 's Pharmaceutical Sciences, 20th Edition, 2000, Merck Publishing Company, Easton, Pennsylvania, which is incorporated herein by reference. A crystalline Form zeta of Ibandronatc sodium of this invention can also be administered in sustained release forms or from sustained release drug delivery systems. A description of representative sustained release materials can also be found in the incorporated materials in Remington's Pharmaceutical Sciences.

While the present invention has been described in terms of its specific embodiments, certain modifications and equivalents will be apparent to those skilled in the art and are included within the scope of the present invention.

In the following, the present invention shall be illustrated by means of some examples, which arc not construed to be viewed as limiting the scope of the invention.

### Example 1

### Preparation of Form ε of Ibandronate sodium

Ibandronate sodium (20 g) was suspended at 20 °C in a round bottom flask containing water (100 mL) and 2-propanol (130 mL). While stirring, the mixture was heated to 85°C until a clear solution was obtained. Then the system was cooled to 15 °C and the resulting solid was filtered, washed with 2-propanol and dried under reduced pressure at 50 °C. Ibandronate sodium Form ε (19 g), having an XRPD pattern as substantially depicted in Figure 1, was obtained.

### Example 2

### Preparation of Form zeta of Ibandronate sodium (hemihydrate)

Ibandronate sodium Form ε (isopropanol 9.5%; KF 2.0%; assay 88.3% on dry base; 42,2 g, 0.1088 mol) was suspended in 210 mL of acetone containing 5-6% v/v of water. The mixture was heated under stirring to reflux temperature (about 56°C) and kept in these conditions for 4-6 hours. The system spontaneously was allowed to cool to room temperature. The solid so obtained was filtered and washed with acetone obtaining wet product (54 g). After drying under reduced pressure at 40-50°C to constant weight Ibandronate sodium hemihydrate Form zeta was afforded (36,0 g; KF 2.7%; 94,1% yield), having an XRPD as substantially depicted respectively in Figure 2.

### Example 3

### Preparation of Form zeta of Ibandronate sodium (hemihydrate)

Ibandronate sodium Form ε (isopropanol 9.5%; KF 2.0%; assay 88.3% on dry base) was refluxed in 2-propanol. After filtration and drying at 135-140 °C under reduced pressure Ibandronate sodium hemihydrate Form zeta was afforded (KF 2.4%;), having an XRPD as substantially depicted respectively in Figure 2.

### Example 4

### Preliminary stability studies of Form zeta of Ibandronate sodium (hemihydrate)

Form zeta of Ibandronate sodium was placed under the following conditions:
- thermal stress at 70 - 75 °C under reduced pressure in an oven
- a nitrogen atmosphere at room temperature
- in a sealed polythene bag at room temperature.

After one week under said conditions, no change was observed in its XRPD patterns.

## Claims

1. A crystalline form of Ibandronate sodium (Form zeta) having an XRPD pattern comprising the following peaks:
| 2θ (degrees) (± 0.2°) | I/Imax (%) |
|---|---|
| | |
| 6,2 | 100 |
| 12,3 | 1 |
| 14,5 | 1 |
| 15,1 | 1 |
| 16,9 | 2 |
| 17,3 | 2 |
| 18,0 | 1 |
| 19,6 | 1 |
| 20,0 | 1 |
| 21,1 | 1 |
| 21,5 | 1 |
| 24,7 | 2 |
| 25,9 | 5 |
| 26,7 | 2 |
| 27,6 | 1 |
| 29,2 | 2 |
| 31,0 | 4 |
| 34,6 | 1 |
| 37,2 | 3 |
wherein said form is a hemihydrate.

2. A crystalline form according to claim 1, **characterized by** a DSC curve showing an endothermic peak at about 172 °C and two close endothermic peaks at about 183 °C and 188 °C.

3. A crystalline form according to claim 1 for use as a medicament.

4. A pharmaceutical composition comprising the crystalline form according to claim 1, optionally together with at least one pharmaceutically acceptable excipient.

5. A crystalline form of Ibandronate sodium (Form ε) having an X-ray powder diffraction pattern wherein the most prominent peaks are as follows:
| 2θ (degrees) (± 0.2°) | I/Imax (%) |
|---|---|
| 4,9 | 100,0 |
| 6,0 | 8,8 |
| 9,9 | 0,5 |
| 11,0 | 0,5 |
| 13,6 | 0,4 |
| 14,0 | 0,5 |
| 16,0 | 0,2 |
| 17,1 | 0,8 |
| 18,6 | 0,9 |
| 19,4 | 0,7 |
| 19,9 | 1,2 |
| 22,1 | 0,4 |
| 23,8 | 0,4 |
| 24,7 | 0,5 |
| 25,9 | 0,7 |
| 26,5 | 0,5 |
| 28,0 | 0,5 |
| 28,4 | 0,6 |
| 30,3 | 0,7 |
| 34,9 | 0,3 |
| 36,3 | 0,7 |
wherein said crystalline form is a hemisolvate of 2-propanol and a hemihydrate.

6. A process for preparing the crystalline form according to claim 5, which comprises the steps of:
a) suspending Ibandronate sodium in a mixture of water and 2-propanol;
b) heating the suspension in order to obtain a clear solution;
c) cooling the solution in order to crystallize a solid;
d) recovering the crystalline form.

7. The process according to claim 6, wherein in step a) water and 2-propanol are in a ratio of about 1:3 to about 1:5 volume/volume.

8. The process according to claim 6, wherein in step a) Ibandronate sodium is in a ratio of about 1:4 to about 1:6 weight/volume with to water.

9. The process according to claim 6, wherein in step b) the suspension is heated to a temperature comprised in a range of about 80 to about 100 °C.

10. A process for preparing the crystalline form according to claim 1, which comprises the step of refluxing in acetone with 5-6% (v/v) of water a crystalline Form ε according to claim 5.

11. A process for preparing the crystalline form according to claim 1, which comprises:
a) refluxing in 2-propanol a crystalline Form ε according to claim 5,
b) cooling the system to precipitate a solid,
c) isolating the solid,
d) drying at a temperature comprised in the range of 135-140°C under reduced pressure to render Form zeta.

## Patentansprüche

1. Kristallform von Ibandronat-Natrium (Zeta-Form) aufweisend einen XRPD-Diagramm umfassend folgende Peaks:
| 2θ (Grad) (± 0,2°) | I/Imax (%) |
|---|---|
| | |
| 6,2 | 100 |
| 12,3 | 1 |
| 14,5 | 1 |
| 15,1 | 1 |
| 16,9 | 2 |
| 17,3 | 2 |
| 18,0 | 1 |
| 19,6 | 1 |
| 20,0 | 1 |
| 21,1 | 1 |
| 21,5 | 1 |
| 24,7 | 2 |
| 25,9 | 5 |
| 26,7 | 2 |
| 27,6 | 1 |
| 29,2 | 2 |
| 31,0 | 4 |
| 34,6 | 1 |
| 37,2 | 3 |
wobei die Form ein Hemihydrat ist.

2. Kristallform nach Anspruch 1, **gekennzeichnet durch** eine DSC-Kurve, die einen endothermischen Peak bei ca. 172 °C und zwei nahe beieinander liegenden endothermischen Peaks bei ca. 183 °C und 188 °C aufweist.

3. Kristallform nach Anspruch 1 zur Verwendung als Arzneimittel.

4. Pharmazeutische Zusammensetzung umfassend eine Kristallform nach Anspruch 1, gegebenenfalls zusammen mit mindestens einem pharmazeutisch akzeptablen Hilfsstoff.

5. Kristallform von Ibandronat-Natrium (ε-Form) aufweisend einen Röntgenpulverdiffraktometrie-Diagramm bei dem die markantesten Peaks diese sind:
| 2θ (Grad) (± 0,2°) | I/Imax (%) |
|---|---|
| 4,9 | 100,0 |
| 6,0 | 8,8 |
| 9,9 | 0,5 |
| 11,0 | 0,5 |
| 13,6 | 0,4 |
| 14,0 | 0,5 |
| 16,0 | 0,2 |
| 17,1 | 0,8 |
| 18,6 | 0,9 |
| 19,4 | 0,7 |
| 19,9 | 1,2 |
| 22,1 | 0,4 |
| 23,8 | 0,4 |
| 24,7 | 0,5 |
| 25,9 | 0,7 |
| 26,5 | 0,5 |
| 28,0 | 0,5 |
| 28,4 | 0,6 |
| 30,3 | 0,7 |
| 34,9 | 0,3 |
| 36,3 | 0,7 |
wobei die Kristallform ein Hemisolvat aus 2-Propanol und ein Hemihydrat ist.

6. Verfahren zur Herstellung der Kristallform nach Anspruch 5 umfassend die folgenden Schritte:
a) Herstellung einer Suspension von Ibandronat-Natrium in einer Mischung aus Wasser und 2-Propanol;
b) Erwärmung der Suspension um eine klare Lösung zu erhalten;
c) Abkühlung der Lösung um einen Feststoff zu kristallisieren;
d) Rückgewinnung der Kristallform.

7. Verfahren nach Anspruch 6, wobei im Schritt a) Wasser und 2-Propanol in einem Verhältnis von ca. 1:3 bis ca. 1:5 (Vol./Vol.) vorhanden sind.

8. Verfahren nach Anspruch 6, wobei im Schritt a) Ibandronat-Natrium in einem Verhältnis von ca. 1:4 bis ca. 1:6 Gew./Vol. zum Wasser vorhanden ist.

9. Verfahren nach Anspruch 6, wobei im Schritt b) die Suspension auf eine Temperatur erwärmt wird, die bei zwischen ca. 80 bis ca. 100 °C liegt.

10. Verfahren zur Herstellung der Kristallform nach Anspruch 1 umfassend den Schritt bei dem eine Kristallform ε nach Anspruch 5 in Aceton mit 5-6% (Vol./Vol.) Wasser refluxiert wird.

11. Verfahren zur Herstellung der Kristallform nach Anspruch 1 umfassend:
a) Rückfluss einer Kristallform ε nach Anspruch 5 in 2-Propanol,
c) Abkühlung des Systems, damit ein Feststoff ausfällt,
c) Isolierung des Feststoffs,
d) Trocknen bei einer Temperatur im Bereich von 135-140 °C und unter vermindertem Druck um die Zeta-Form abzugeben.

## Revendications

1. Forme cristalline de sodium d'Ibandronate (Forme Zêta) ayant un diagramme de XRPD comprenant les pics suivants:
| 2θ (degrés) (± 0,2°) | I/Imax (%) |
|---|---|
| | |
| 6,2 | 100 |
| 12,3 | 1 |
| 14,5 | 1 |
| 15,1 | 1 |
| 16,9 | 2 |
| 17,3 | 2 |
| 18,0 | 1 |
| 19,6 | 1 |
| 20,0 | 1 |
| 21,1 | 1 |
| 21,5 | 1 |
| 24,7 | 2 |
| 25,9 | 5 |
| 26,7 | 2 |
| 27,6 | 1 |
| 29,2 | 2 |
| 31,0 | 4 |
| 34,6 | 1 |
| 37,2 | 3 |
étant ladite forme un hémihydrate.

2. Forme cristalline selon la revendication 1 **caractérisée par** une courbe de DSC qui présente un pic endothermique à environ 172 °C et deux pics endothermiques proches l'un de l'autre à environ 183 °C et 188 °C.

3. Forme cristalline selon la revendication 1 pour utilization comme médicament.

4. Composition pharmaceutique comprenant la forme cristalline selon la revendication 1, facultativement avec au moins un excipient pharmaceutiquement acceptable.

5. Forme cristalline de sodium d'Ibandronate (Forme ε) ayant un diagramme de diffraction de rayons X sur poudre dans lequel les pics les plus marqués sons les suivants:
| 2θ (degrés) (± 0,2°) | I/Imax (%) |
|---|---|
| 4,9 | 100,0 |
| 6,0 | 8,8 |
| 9,9 | 0,5 |
| 11,0 | 0,5 |
| 13,6 | 0,4 |
| 14,0 | 0,5 |
| 16,0 | 0,2 |
| 17,1 | 0,8 |
| 18,6 | 0,9 |
| 19,4 | 0,7 |
| 19,9 | 1,2 |
| 22,1 | 0,4 |
| 23,8 | 0,4 |
| 24,7 | 0,5 |
| 25,9 | 0,7 |
| 26,5 | 0,5 |
| 28,0 | 0,5 |
| 28,4 | 0,6 |
| 30,3 | 0,7 |
| 34,9 | 0,3 |
| 36,3 | 0,7 |
étant ladite forme cristalline un hémisolvate de 2-propanol et d'un hémihydrate.

6. Procédé de préparation de la forme cristalline selon la revendication 5 comprenant les étapes de:
a) former une suspension de sodium d'Ibandronate dans un mélange d'eau et 2-propanol;
b) échauffer la suspension afin d'obtenir une solution claire;
c) refroidir la solution afin de cristalliser un solide;
d) récupérer la forme cristalline.

7. Procédé selon la revendication 6 dans lequel dans l'étape a) de l'eau et du 2-propanol sont présentes dans un rapport d'environ 1:3 à environ 1:5 volume/volume.

8. Procédé selon la revendication 6 dans lequel dans l'étape a) le sodium d'Ibandronate est présente dans un rapport d'environ 1:4 à environ 1:6 poids/volume avec l'eau.

9. Procédé selon la revendication 6 dans lequel dans l'étape b) la suspension est échauffée à une température comprise dans l'intervalle allant d'environ 80 à environ 100 °C.

10. Procédé de préparation de la forme cristalline selon la revendication 1 comprenant l'étape de refluxer dans acétone avec 5-6% (v/v) d'eau une forme cristalline ε selon la revendication 5.

11. Procédé de préparation de la forme cristalline selon la revendication 1 comprenant:
a) refluxer dans 2-propanol une forme cristalline ε selon la revendication 5,
b) refroidir le système afin de faire précipiter un solide,
c) isoler le solide,
d) sécher à une température comprise dans l'intervalle de 135-140 °C sous pression réduite afin de fournir la Forme Zêta.
